# EUROPEAN PATENT APPLICATION

(11) **EP 1 310 484 A1**
(43) Date of publication of application: **14.05.2003**
(21) Application number: 01955697.6
(22) Date of filing: 13.08.2001
(51) Int. Cl.: C07C 401/00, A61K 31/593, A61P 19/10

(54) **1-METHYL-20-EPIVITAMIN D DERIVATIVE**

(30) Priority: 14.08.2000 JP 2000245607
(71) Applicant: CHUGAI SEIYAKU KABUSHIKI KAISHA, Tokyo, 115-8543 (JP)
(72) Inventor: TAKAYAMA, Hiroaki, Shibuya-ku, Tokyo 151-0072 (JP); FUJISHIMA, Toshie, Hachioji-shi, Tokyo 193-0834 (JP)
(74) Representative: HOFFMANN - EITLE
(86) International application number: JP0106977
(87) International publication number: WO02014268

(57) **Abstract**

The object of the present invention is to synthesize vitamin D derivatives in which the 1-position is substituted with methyl and the steric configuration at the 20-position is epimerized.

The present invention provides vitamin D derivatives of Formula (1): 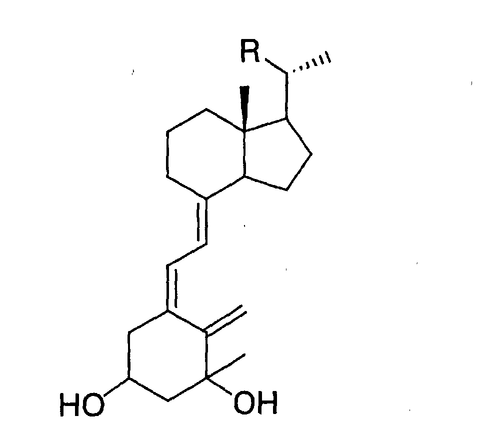 wherein R is straight or branched alkyl optionally substituted with hydroxy.

## Description

### TECHNICAL FIELD

The present invention relates to novel vitamin D derivatives, more particularly, relates to 1-methyl-20-epi-vitamin D derivatives, in which the steric configuration at the 20-position is not native and the 1-position is substituted with methyl.

### BACKGROUND ART

Active vitamin D derivatives including 1α,25-dihydroxyvitamin D₃ are known to have many physiological activities such as calcium metabolism regulatory activities, growth inhibitory and differentiation inducing activities for tumor cells and immunoregulatory activities. However, some active vitamin D₃ derivatives may cause hypercalcemia during long-term and continuous administration, therefore they are not suitable for use as antitumor agents, antirheumatic agents and the like. Thus, a number of synthetic studies have been conducted to obtain such vitamin D derivatives that are excellent in specific activities among the above-mentioned activities.

For example, if the A-ring of an active vitamin D₃ derivative is substituted, the possible conformation of the molecule may be limited, resulting in a characteristic activity of the resulting vitamin D derivative. For example, 1α,25-dihydroxyvitamin D₃ derivatives having methyl at the 2- or 4-position are described by K. Konno et al. (Bioorg. Med. Chem. Lett., 1998, 8, 151) and T. Fujishima et al. (ibid., 1998, 8, 2145) and in Abstracts of the 118th Annual Meeting of the Pharmaceutical Society of Japan 2 (p.171). In addition, a vitamin D derivative having methyl at the 1-position is described in Abstracts of the 120th Annual Meeting of the Pharmaceutical Society of Japan 2 (p.105). However, no vitamin D₃ derivative has been reported in which the 1-position is substituted with methyl and the steric configuration at the 20-position is epimerized.

### DISCLOSURE OF THE INVENTION

An object of the present invention is to provide and to synthesize 1-methyl-20-epi-vitamin D derivatives. Another object of the present invention is to evaluate biological activity of the resulting 1-methyl-20-epi-vitamin D derivatives.

As a result of careful studies so as to achieve the above mentioned objects, the inventors of the present invention have succeeded in synthesizing desired vitamin D derivatives by coupling A-ring part precursors and CD-ring parts using palladium catalyst after synthesizing the A-ring part precursors and the CD-ring parts separately by the method described in Abstracts of the 120th Annual Meeting of the Pharmaceutical Society of Japan 2 (p.105) and by the method described by T. Fujishima et al. (Bioorg. Med. Chem., 2000, 8, 123), respectively; thereby they achieved the present invention.

According to one aspect of the present invention, there is provided a vitamin D derivative of Formula (1): 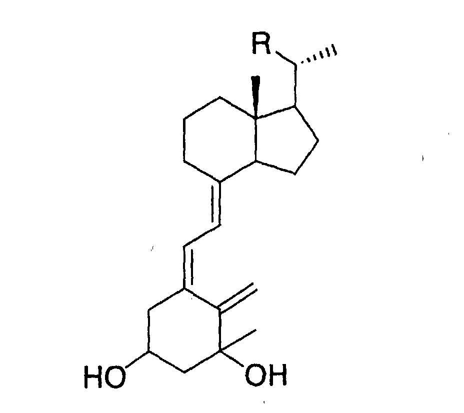 wherein R is straight or branched alkyl optionally substituted with hydroxy.

For R of Formula (1), straight or branched C₁₋₁₂ alkyl substituted with hydroxy is preferred and straight or branched C₁₋₁₀ alkyl substituted with hydroxy is more preferred.

Particularly preferably, R is 4-hydroxy-4-methylpentyl or 4-ethyl-4-hydroxyhexyl, more preferably R is 4-hydroxy-4-methylpentyl.

The vitamin D derivatives of the present invention may be used for medicines, for example, for the purpose of calcium metabolism regulating agent and the like.

Therefore, according to the present invention, there is provided a pharmaceutical composition comprising the vitamin D derivative of Formula (1) as an active ingredient.

Furthermore, there is provided use of the vitamin D derivative of Formula (1) as a medicine.

The vitamin D derivatives of the present invention can be also used as test reagents in studying the metabolism of active vitamin D₃ (i.e., 1α,25-dihydroxyvitamin D₃).

The contents of the specification of Japanese Patent Application No. 2000-245607, the application on the basis of which the present application claims priority are to be incorporated in their entirety by reference.

### PREFERRED MODE FOR CARRYING OUT THE INVENTION

Detailed modes and methods with respect to vitamin D derivatives of Formula (1) of the present invention are described in further detail below.

In the present specification, "straight or branched alkyl" is preferably straight or branched C₁₋₁₅ alkyl; examples thereof include methyl, ethyl, n-propyl, i-propyl, n-butyl, s-butyl, i-butyl and t-butyl, and further include pentyl, hexyl, heptyl, octyl, nonyl, decanyl, etc.

"Straight or branched alkyl optionally substituted with hydroxy" means that one or more hydrogen atoms of the above-mentioned straight or branched alkyl may be substituted with hydroxy. In the definition of R, the number of hydrogen atoms substituted with hydroxy is preferably 1, 2 or 3, more preferably 1 or 2 and most preferably 1.

Non-limiting examples of R include 4-hydroxy-4-methylpentyl, 4-ethyl-4-hydroxyhexyl, 6-hydroxy-6-methyl-2-heptyl, 7-hydroxy-7-methyl-2-octyl, 5,6-dihydroxy-6-methyl-2-heptyl, 4,6,7-trihydroxy-6-methyl-2-heptyl, etc.

Preferably R is straight or branched C₁₋₁₂ alkyl substituted with hydroxy, more preferably straight or branched C₃₋₁₀ alkyl substituted with hydroxy. Further more preferably R is 4-hydroxy-4-methylpentyl or 4-ethyl-4-hydroxyhexyl and most preferably R is 4-hydroxy-4-methylpentyl.

The vitamin D derivatives of Formula (1) of the present invention can be used as active ingredients of pharmaceutical compositions (such as a calcium metabolism regulating agent).

Although there is no limitation with respect to methods of synthesizing vitamin D derivatives of Formula (I) of the present invention which are novel compounds, they are synthesizable, for example, by synthesizing A-ring and CD-ring parts of the vitamin D derivatives separately and then coupling them together, as described in the following Examples.

CD-ring part compounds of vitamin D derivatives are known. Alternatively, a desired CD-ring compound is obtainable by appropriately modifying a side chain of a known CD-ring compound or is obtainable from a known vitamin D derivative having a corresponding side chain.

Examples of such a known vitamin D derivative include those which are disclosed in Japanese Patent Publication (Kokai) Nos. 61-267550 A, 6-72994 A and 6-256300 A and Japanese Patent Publication (Kohyo) Nos. 4-503669 A, 4-504573 A and 10-182597 A, WO94/14766, WO95/27697, etc.

According to Scheme 4 described by T. Fujishima et al (Bioorg. Med. Chem., 2000, 8, 123), a CD-ring compound having a desired side chain is obtainable as follows:
an aldehyde led from the ozonolysis of vitamin D₂ is treated with a base to epimerize the stereochemistry on a carbon, the position of which corresponds to the 20-position of the steroid skeleton. A desired side chain is introduced to the epimerized aldehyde to give a protected alcohol, which is then deprotected and oxidized. Thus obtained ketone is converted to a bromomethylene to give a CD-ring compound having the desired side chain.

An A-ring compound having methyl at the 1-position is synthesizable by the method described on page 105 of Abstracts of the 120th Annual Meeting of the Pharmaceutical Society of Japan 2 via a 3-methylbutane-1,2,4-triol derivative, which is synthesizable from 3-methylbut-3-en-1-ol, as a starting material; however there is no limitation with respect to a method for synthesizing the compounds.

An A-ring compound and a CD-ring compound can be coupled by a known conventional method. Namely, an A-ring compound, which is obtainable by the above method and which has a triple bond at one terminal and a double bond at the other terminal, is reacted with a CD-ring compound, which has bromomethylene at the coupling site for the A-ring compound, in the presence of a palladium catalyst in an appropriate solvent.

After the coupling reaction, the resulting product is purified in a usual manner such as thin layer chromatography and subjected to removal of the hydroxy protecting groups, to give a desired vitamin D derivative.

When the compounds of the present invention are used as medicines, they are preferably formulated into appropriate dosage forms with pharmaceutically acceptable carriers, excipients, disintegrants, lubricants, binders, flavors, colorants and the like; examples of the dosage forms include tablets, granules, fine granules, capsules, powders, injections, solutions, suspensions, emulsions, percutaneous administration formulations, suppositories and the like.

There is no restriction on routes of administration for the compounds of the present invention as medicines; they may be administered orally or parenterally (intravenously, intramuscularly, intraperitoneally, percutaneously and the like).

Dosage of compounds of the present invention as medicines can be appropriately chosen depending on target disease, conditions, body type, constitution, age and sex of the patient, administration route, dosage form and other factors. Typically, the lower limit for an adult ranges from 0.001 µg to 0.1 µg and preferably around 0.01 µg daily, and the upper limit for an adult ranges from 100 µg to 10000 µg and preferably from 200 µg to 1000 µg daily, which may be administered at a time or in divided portions two or three times a day.

### EXAMPLES

The present invention will be described specifically by way of the following Examples, which in no way limit the invention. The following schemes show the reactions carried out in Examples.

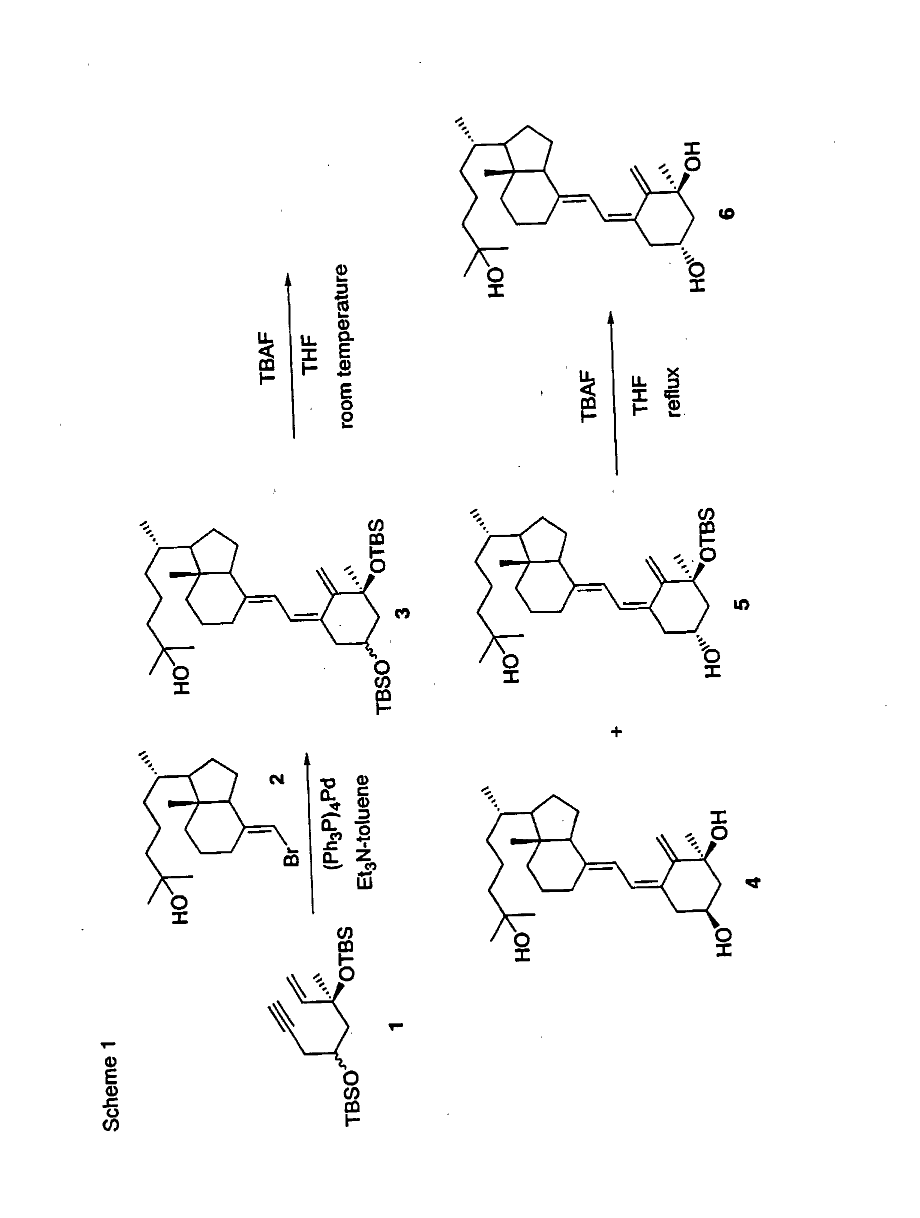

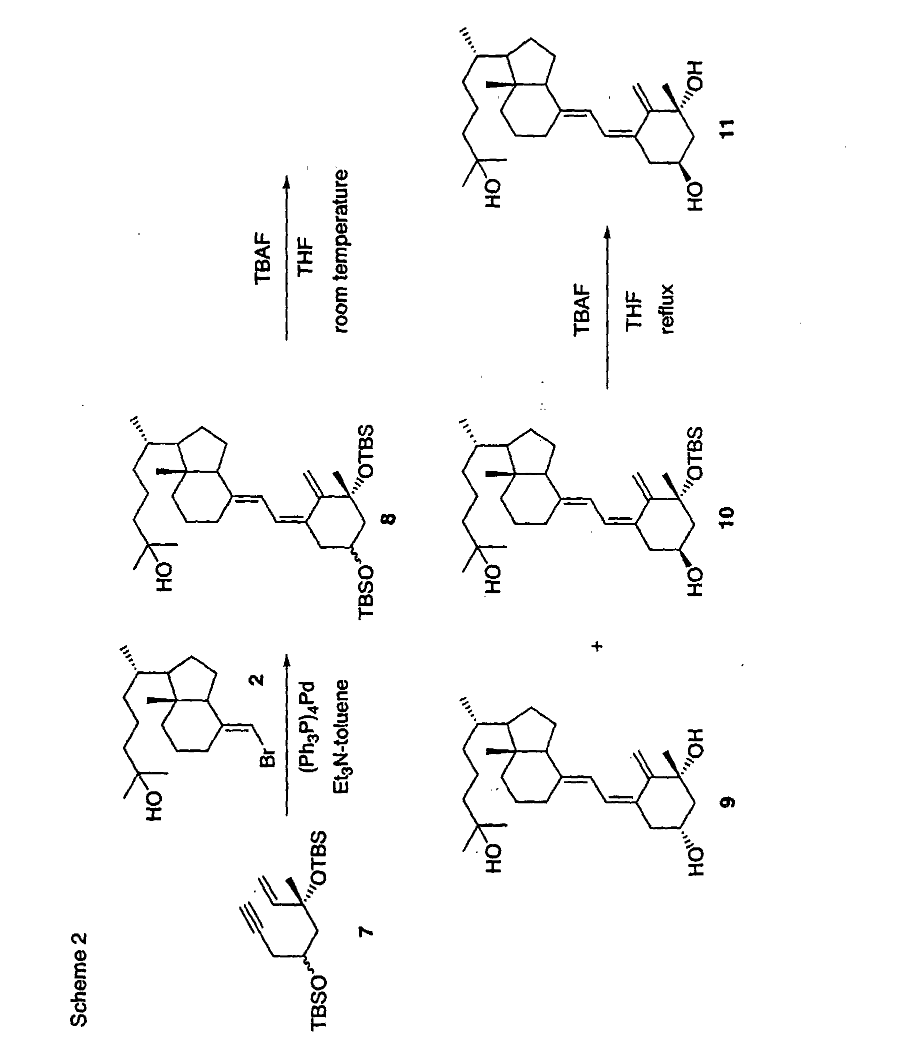

### (Example 1) Synthesis of (5Z, 7E)-(1S,3S,20S)-1-Methyl-9,10-seco-5,7,10(19)-cholestatriene-1,3,25-triol (Compound 4)

(E)-de-A,B-8-(bromomethylene)cholestan-25-ol (Compound 2) (90 mg, 0.25 mmol) and triethylamine (3 ml) were mixed in toluene (2 ml); the resulting solution was mixed with (Ph₃P)₄Pd (145 mg, 0.13 mmol) and stirred at room temperature for 10 minutes. A solution of an A-ring compound (Compound 1) (150 mg, 0.39 mmol) was then added, followed by stirring at room temperature for a further 20 minutes. The reaction mixture was heated under reflux for 4 hours and filtered through a pad of silica gel with ethyl acetate. After evaporation of the solvent, the crude product was purified by silica gel preparative thin layer chromatography (ethyl acetate:n-hexane = 1:3) to give Compound 3 (63 mg) as a colorless foam in 38% yield.

The above-mentioned Compound 3 (63 mg, 0.096 mmol) which was a protected vitamin D derivative was dissolved in THF (2 ml). While stirring thus obtained solution at 0°C under an argon atmosphere, TBAF (tetrabutylammonium fluoride) (1.0 M solution in THF, 0.5 ml, 0.5 mmol) was added. The reaction mixture was stirred at room temperature for 6 hours, mixed with brine, and extracted with ethyl acetate. The organic layer was dried over sodium sulfate and filtered. The filtrate was evaporated to remove the solvent and thus obtained crude product was purified by silica gel preparative thin layer chromatography (ethyl acetate:n-hexane = 1:1) to give Compound 4 (12 mg, 30%) and Compound 5 (11 mg, 21%), each as a white solid. Compound 4 was further purified by reverse phase recycle HPLC (YMC-Pack ODS column, 20 mm X 150 mm, 9.0 ml/min, acetonitrile:water = 8:2) for biological activity evaluation.
UV (EtOH) λmax 266 nm, λmin 226 nm; ¹H NMR (400 MHz, CDCl₃) δ 0.55 (3 H, s), 0.85 (3H, d, J = 6.4 Hz), 1.19 (6 H, s), 1.23 (3 H, s), 1.80 (1 H, dd, J = 14.3, 3.4 Hz), 2.11 (1 H, ddd, J = 14.3, 3.1, 2.4 Hz), 2.40 (2H, m), 2.84 (1 H, dd, J = 11.3. 3.7 Hz), 4.42 (1 H, t, J = 3.1 Hz), 5.02 (1 H, d, J = 2.1 Hz), 5.28 (1 H, d, J = 2.1 Hz), 6.07 (1 H, d, J = 11.3 Hz), 6.45 (1 H, d, J = 11.3 Hz); MS 430 [M]⁺, 412 [M-H₂O]⁺; HRMS calcd. for [C₂₈H₄₆O₃] 430.3447, found 430.3448.

### (Example 2) Synthesis of (5Z, 7E)-(lS,3R,20S)-1-Methyl-9,10-seco-5,7,10(19)-cholestatriene-1,3,25-triol (Compound 6)

Under an argon atmosphere at 60°C, a solution of Compound 5 (11 mg, 0.02 mmol) in THF (3 ml) was treated with TBAF (1.0 M solution in THF, 0.2 ml, 0.2 mmol) for 24 hours. After the treatment, thus obtained mixture was mixed with brine and extracted with ethyl acetate. The organic layer was dried over sodium sulfate, and filtered. The filtrate was evaporated for removing the solvent to give a crude product, which was then subjected to silica gel preparative thin layer chromatography (ethyl acetate:n-hexane = 2:1) to give Compound 6 (3.8 mg) as a white solid in 44% yield. Compound 6 was further purified by reverse phase recycle HPLC (YMC-Pack ODS column, 20 mm X 150 mm, 9.0 ml/min, acetonitrile:water = 8:2) for biological activity evaluation.
UV (EtOH) λmax 263 nm, λmin 228 nm; ¹H NMR (400 MHz, CDCl₃) δ 0.55 (3 H, s), 0.85 (3H, d, J = 6.4 Hz), 1.21 (6 H, s), 1.32 (3 H, s), 1.51 (1 H, dd, J = 12.2, 11.6 Hz), 2.19 (1 H, ddd, J = 12.8, 5.5, 2.7 Hz), 2.24 (1 H, dd, J = 14.0, 2.4 Hz), 2.42 (1 H, d, J = 13.7 Hz), 2.81 (1 H, m), 4.34 (1 H, ddt, J = 11.3, 5.2, 2.2 Hz), 5.02 (1 H, t, J = 1.8 Hz), 5.38 (1 H, t, J = 1.8 Hz), 6.05 (1 H, d, J = 11.6 Hz), 6.32 (1 H, dd, J = 11.0, 1.8 Hz); MS 430 [M]⁺, 412 [M-H₂O]⁺; HRMS calcd. for [C₂₈H₄₆O₃] 430.3447, found 430.3447.

### (Example 3) Synthesis of (5Z,7E)-(1R,3R,20S)-1-Methyl-9,10-seco-5,7,10(19)-cholestatriene-1,3,25-triol (Compound 9)

(E)-de-A,B-8-(bromomethylene)cholestan-25-ol (Compound 2) (90 mg, 0.25 mmol) and triethylamine (3 ml) were dissolved in toluene (2 ml), to which (Ph₃P)₄Pd (145 mg, 0.13 mmol) was added and stirred for 10 min at room temperature. Then a solution of Compound 7 (192 mg, 0.50 mmol) which was an A-ring part compound in toluene (2 ml) was added to the mixture, followed by stirring for a further 20 minutes at room temperature. The mixture was heated under reflux for 4 hours, the reaction mixture was filtered through a pad of silica gel with ethyl acetate. After evaporation of the solvent, the resulting crude product was purified by silica gel preparative thin layer chromatography (ethyl acetate:n-hexane = 1:3) to give Compound 8 (96 mg) as a colorless foam in 58% yield.

TBAF (1.0 M solution in THF, 0.7 ml, 0.7 mmol) was added to a stirred solution of Compound 8 (96 mg, 0.15 mmol) which was a protected vitamin D derivative in THF (2 ml) under an argon atmosphere at 0°C. The reaction mixture was stirred at room temperature for 6 hours, mixed with brine, and extracted with ethyl acetate. The organic layer was dried over sodium sulfate, and filtered. The filtrate was evaporated to remove the solvent, and thus obtained crude product was separated by silica gel preparative thin layer chromatography (ethyl acetate:n-hexane = 1:1) to give Compound 9 (22 mg, 34%) and Compound 10 (17 mg, 21%), each as a white solid. Compound 9 was further purified by reverse phase recycle HPLC (YMC-Pack ODS column, 20 mm X 150 mm, 9.0 ml/min, acetonitrile:water = 8:2) for biological activity evaluation.
UV (EtOH) λmax 265 nm, λmin 227 nm; ¹H NMR (400 MHz, CDCl₃) δ 0.53 (3 H, s), 0.84 (3 H, d, J = 6.4 Hz), 1.21 (6 H, s), 1.32 (3 H, s), 1.81 (1 H, dd, J = 14.4, 3.4 Hz), 2.09 (1 H, m), 2.40 (2 H, m), 2.84 (1 H, dd, J = 11.9, 4.0 Hz), 4.39 (1 H, t, J = 3.1 Hz), 4.98 (1 H, d, J = 1.8 Hz), 5.26 (1 H, d, J = 1.8 Hz), 6.02 (1 H, d, J = 11.3 Hz), 6.44 (1 H, d, J = 11.0 Hz); MS 430 [M]⁺, 412 [M-H₂O]⁺, HRMS calcd. for [C₂₈H₄₆O₃] 430.3447, found 430.3465.

### (Example 4) Synthesis of (5Z,7E)-(1R,3S,20S)-1-Methyl-9,10-seco-5,7,10(19)-cholestatriene-1,3,25-triol (Compound 11).

Under an argon atmosphere at 60°C, a solution of Compound 10 (17 mg, 0.03 mmol) in THF (3 ml) was treated with TBAF (1.0 M solution in THF, 0.4 ml, 0.4 mmol) for 24 hours. After the treatment, thus obtained mixture was mixed with brine and extracted with ethyl acetate. The organic layer was dried over sodium sulfate and filtered. The filtrate was evaporated for removing the solvent to give a crude product, which was then subjected to silica gel preparative thin layer chromatography (ethyl acetate:n-hexane = 2:1) to give Compound 11 (4.8 mg) as a white solid in 37% yield. Compound 21 was further purified by reverse phase recycle HPLC (YMC-Pack ODS column, 20 mm X 150 mm, 9.0 ml/min, acetonitrile:water = 8:2) for biological activity evaluation.
UV (EtOH) λmax 263 nm λmin 228 nm; ¹H NMR (400 MHz, CDCl₃) δ 0.53 (3 H, s), 0.85 (3H, d, J = 6.7 Hz), 1.21 (6 H, s), 1.30 (3 H, s), 1.53 (1 H, dd, J = 12.5, 11.0 Hz), 2.18 (1 H, ddd, J = 12.5, 5.2, 2.4 Hz), 2.25 (1 H, dd, J = 13.7, 2.1 Hz), 2.41 (1 H, d, J = 13.7 Hz), 2.81 (1 H, dd, J = 12.2, 4.0 Hz), 4.36 (1 H, ddt, J = 11.0, 5.2, 1.8 Hz), 4.97 (1 H, m), 5.36 (1 H, t, J = 1.8 Hz), 6.10 (1 H, d, J = 11.3 Hz), 6.32 (1 H, dd, J = 11.3 Hz); MS 430 [M]⁺, 412 [M-H₂O]⁺; HRMS calcd. for [C₂₈H₄₆O₃] 430.3447, found 430.3444.

### (Test example) Assay for binding to bovine thymus vitamin D receptor (VDR)

Bovine thymus 1α,25-dihydroxyvitamin D₃ receptor was purchased from Yamasa Biochemcal and, just before use, one ampule (approximately 25 mg) of the receptor was dissolved in 55 mL of 0.05 M phosphate buffer (pH 7.4) containing 0.3M KCl and 5 mM dithiothreitol to prepare a receptor solution. Compounds 4, 6, 9 and 11 synthesized in the above Examples 1 to 4 were used as test compounds, and 1α,25-dihydroxyvitamin D₃ was used as standard.

Ethanol solutions of the test compounds and 1α,25-dihydroxyvitamin D₃ were prepared at various concentrations. Each of the ethanol solutions (50 µl) of the test compounds, and 1α,25-dihydroxyvitamin D₃ was pre-incubated at 25°C for 1 hour with 500 µl (0.23 mg protein) of the receptor solution. [³H]-1α,25-Dihydroxyvitamin D₃ was added to the pre-incubated solution at the final concentration of 0.1 nM, followed by incubation overnight at 4°C. Each of the reaction mixtures was treated with dextran coated charcoal for 30 minutes at 4°C and centrifuged at 3000 rpm for ten minutes to separate the bound and free forms of [³H]-1α,25-dihydroxyvitamin D₃. Each of the resultant supernatants (500 µl) was mixed with ACS-II (9.5 ml) (AMERSHAM, England) for radioactivity measurement.

The VDR binding properties of the test compounds expressed in relative value with that of 1α,25-dihydroxyvitamin D₃ taken as 100 were shown in Table below.

**Table**

| Compound | Compound 4 | Compound 6 | Compound 9 | Compound 11 |
|---|---|---|---|---|
| VDR binding properties | 1 | 1 | 0.017 | 0.004 |

### INDUSTRIAL APPLICABILITY

As described above, the vitamin D derivatives of the present invention are novel, exhibit excellent physiological activities, and are expected to be useful as medicines, for example, for calcium metabolism regulation. The compounds of the present invention may be useful as reagents for studying metabolism of active vitamin D₃ (i.e., 1α,25-dihydroxyvitamin D₃).

## Claims

1. A vitamin D derivative of Formula (1): 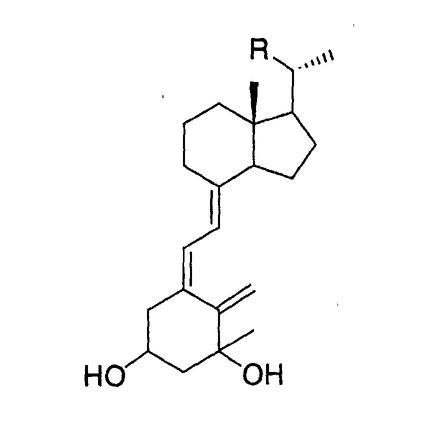 wherein R is straight or branched alkyl optionally substituted with hydroxy.

2. The vitamin D derivative of claim 1 wherein R is straight or branched C₁₋₁₂ alkyl substituted with hydroxy.

3. The vitamin D derivative of claim 1 wherein R is straight or branched C₁₋₁₀ alkyl substituted with hydroxy.

4. The vitamin D derivative of claim 1 wherein R is 4-hydroxy-4-methylpentyl or 4-ethyl-4-hydroxyhexyl.

5. The vitamin D derivative of claim 1 wherein R is 4-hydroxy-4-methylpentyl.

6. A pharmaceutical composition comprising the vitamin D derivative of one of claims 1 to 5 as an active ingredient.
